## Europäisches Patentamt

## European Patent Office

## Office européen des brevets

(19)

(11) Veröffentlichungsnummer: **0 039 044**
**B1**

(12) **EUROPÄISCHE PATENTSCHRIFT**

(45) Veröffentlichungstag der Patentschrift:
21.08.85

(51) Int. Cl.⁴: **A 61 M 5/14**

(21) Anmeldenummer: 81103027.9

(22) Anmeldetag: 22.04.81

(54) Vorrichtung zur intermittierenden Applikation flüssiger Arzneimittel.

(30) Priorität: 24.04.80 DE 3015777

(43) Veröffentlichungstag der Anmeldung:
04.11.81 Patentblatt 81/44

(45) Bekanntmachung des Hinweises auf die Patenterteilung:
21.08.85 Patentblatt 85/34

(84) Benannte Vertragsstaaten:
AT BE CH DE FR GB IT LI LU NL SE

(56) Entgegenhaltungen:
EP - A - 0 019 817
DE - A - 2 651 962
DE - A - 2 920 975
GB - A - 2 011 652

MEDICAL PROGRESS THROUGH TECHNOLOGY, Band
5, Nr. 4, Mai 1978, Seiten 187-193 Springer-Verlag A.M.
ALBISSER et al.: "A portable precision pumping system
for chronic, programmed insulin infusion"
MEDICAL PROGRESS THROUGH TECHNOLOGY, Band
8, Nr. 1, Dezember 1980, Seiten 49-55 Springer-Verlag
C.A. CARLSON et al.: "A portable insulin infusion
system with a rotary solenoid-driven peristaltic pump"
MEDICAL PROGRESS THROUGH TECHNOLOGY, Band
7, Nr. 1, April 1980, Seiten 45-55, Springer-Verlag (Am
14.5.1980 bei EPA zugänglich) W.J. SPENCER et al.:
"Modified hospital pumps for pulsed insulin delivery"

(73) Patentinhaber: **Ferring Biotechnik GmbH,
Wittland 11-13, D-2300 Kiel 1 (DE)**

(72) Erfinder: **Eschweiler, Wilhelm, Borstenkamp 61,
D-2301 Rammsee (DE)**
Erfinder: **Leyendecker, Gerhard, Prof. Dr.,
Clemens-August-Strasse 9, D-5300 Bonn -
Bad-Godesberg (DE)**
Erfinder: **Paulsen, Otto, Dr., Wittland 11,
D-2300 Kiel 1 (DE)**

(74) Vertreter: **Gille, Christian, Dipl.-Ing. et al, Redies ,
Redies, Türk & Gille Bruckner Strasse 20,
D-4000 Düsseldorf 13 (DE)**

## Beschreibung

Die Erfindung betrifft eine Vorrichtung zur intermittierenden Applikation flüssiger Arzneimittel, mit einem auswechselbaren Vorratsbehälter für das Arzneimittel, einem an den Vorratsbehälter angeschlossenen Schlauch zum Abgeben des Arzneimittels, so daß der Vorratsbehälter mit dem Schlauch eine konfektionierte Einheit bildet, einer auf den Schlauch einwirkenden Rollenpumpe, einem batteriegetriebenen Antriebsmotor für die Rollenpumpe, der mit einer Untersetzung versehen ist, und mit einer Steuerung für den Antriebsmotor, die einen Zeitgeber für Impuls- und Pausenzeiten aufweist.

Bei Patientinnen mit hypothalamischer und hyperprolactinamischer Amenorrhöe werden die besten Behandlungsergebnisse erzielt, wenn in einem Abstand von 90 Minuten zwischen Einzeldosen die Pulsation der natürlichen Sekretion von LH-RH simuliert wird, um die Ovulation einzuleiten.

Zum dosierten Zuführen flüssiger und/oder gasförmiger Medikamente sind mit elektrischen Motoren angetriebene Geräte bekannt, welche mit Vorrichtungen für austauschbare Programme versehen sind. Ein mechanisch gesteuertes Gerät für Dauerinfusion ist in der AT-B-210 558 beschrieben. Bei dem aus der DE-B-1 491 747 bekannten Injektionsgerät für einmalige Injektion eines Röntgenkontrastmittels kann die injizierte Menge, die Geschwindigkeit und ggf. eine zeitliche Verzögerung mit einem elektrischen Programmgeber gesteuert werden.

Für die Diabetes-Therapie ist eine Applikationsvorrichtung bekannt, welche eine programmierbare Steuerung eines Elektromotors zum Betätigen einer Injektionsspritze über einen längeren Zeitraum aufweist (DE-B-2 451 424). Bei einer anderen für die Diabetes-Therapie bekannten Infusionsvorrichtung (DE-A-2 758 368) sind ein externes Programmiergerät, eine elektrische Steuervorrichtung und eine Mikrodosiereinheit vorgesehen. Es handelt sich jeweils um Vorrichtungen mit Langzeitprogrammierung und ggf. mit Infusionsratenschaltern.

Auch sind tragbare Infusionsgeräte mit einem auswechselbaren Vorratsbehälter für die Flüssigkeit und einer Rollenpumpe als Förder- und Dosiereinheit zum Fördern der Infusionsflüssigkeit aus dem Vorratsbehälter zur Auslauföffnung eines Katheters bekannt (DE-A-2 920 975, 2 652 026 und 2 651 962). Diese bekannten Geräte sind beispielsweise sehr klein und leichtgewichtig ausgeführt, daß sie entweder implantierbar oder ohne Behinderung extrakorporal am Körper des Patienten tragbar sind. Da sie für die Diabetes-Therapie verwendet werden sollen, ist die infundierbare Flüssigkeitsmenge in unterschiedlichen Raten programmierbar und im Rhythmus von Tagen steuerbar, wobei die Fließraten in der Größenordnung von Mikrolitern pro Stunde ($10^{-6}$ l/h) liegen. Diese Infusionsgeräte (DE-A-2 920 975) weisen die gattungsbildenden Merkmale der vorliegenden Erfindung auf, ohne jedoch durch ihre Steuerung für die intermittierende Applikation von Arzneimitteln geeignet zu sein. Vielmehr ist die Vorrichtung zur kontinuierlichen Applikation flüssiger Arzneimittel, insbesondere von Insulin, bestimmt. Sofern der Basalrate der Infusion dabei ein Aufsetzprogramm zur Erhöhung der Dosis zugeschaltet werden kann, muß dieses eine bestimmte Zeitdauer währende Programm vom Patienten selbst von Hand eingestellt werden. Das Auswechseln des Vorratsbehälters ist bei dieser Vorrichtung relativ umständlich und erfordert es, daß der mit der Rollenpumpe zusammenwirkende Teil des Katheters oder Schlauches zwischen den Kopf der Rollenpumpe und einen Feststehenden Gegenbacken eingelegt werden muß. Aus diesen Gründen sind die genannten Infusionsvorrichtungen zur intermittierenden pulsatorischen Applikation flüssiger Arzneimittel, insbesondere von LH-RH, für die Praxis kaum geeignet.

Eine weitere Applikationsvorrichtung für eine kontinuierliche Infusion flüssiger Arzneimittel weist eine Steuerung für den Antriebsmotor einer Rollenpumpe auf, die einen Zeitgeber mit einem Taktgenerator, einem Frequenzteiler und einer Treiberstufe in intergrierter Schaltung aufweist (Medical Progress through Technology Vol. 8, No. 1, 1980, S. 49—56). Hierbei ist nur die Variation der Impulsfrequenz der Pumpe mittels einer entsprechenden Steuerung eines die Rollenpumpe über ein Sperrklinkengetriebe schrittweise antreibenden Gleichstromantriebes vorgesehen. Eine einfache Variationsmöglichkeit besteht hierbei nur zwischen zwei verschiedenen Förderraten, die vorgewählt werden müssen, wobei die jeweils höhere Förderrate zum Start jedesmal von Hand angesteuert werden muß und nach einer ebenfalls vorzuwählenden Periode automatisch oder bereits vorzeitig von Hand beendet wird. Diese kontinuierliche Applikationsmöglichkeit beinhaltet damit die Gefahr von Bedienungsfehlern und läßt eine automatische zyklische Steuerung der Zugabe nicht zu.

Eine ähnliche Applikationsvorrichtung mit den gattungsbildenden Merkmalen der vorliegenden Erfindung für die Infusion von Insulin zeigt erstmals die Möglichkeit eines intermittierenden Betriebes (Medical Progress through Technology, Vol. 5, No. 4, 1978, S. 187—193), jedoch liegt auch hierbei eine kontinuierliche Applikation als Aufgabe zugrunde, bei der der Einstellbereich der Impulsrate — also der Gesamtmenge des zuzugebenden Infusionsmittels in einer bestimmten Zeitspanne — in Richtung zu kleineren Mengen hin erweitert werden soll, ohne die Konzentration des Arzneimittels herabsetzen zu müssen. Der intermittierende Betrieb wird demnach nur für den Fall vorgeschlagen, in welchem die gewünschte Infusionsmenge bei kontinuierlicher Förderung in Folge der vorgegebenen Förderrate der Pumpe überschritten wird. Eine automatische zyklische Veränderung der Infusionsrate ist nicht vorgesehen oder möglich.

Der Erfindung liegt die Aufgabe zugrunde, eine Vorrichtung zur intermittierenden Applikation flüssiger Arzneimittel zu schaffen, welche die Arzneimittel, insbesondere LH-RH, pulsatorisch in vorgegebenen zeitlichen Abständen mittels einer elektrisch angetriebenen Rollenpumpe aus einem auswechselbaren Vorratsbehälter über ein Ausflußkatheter beispielsweise in die Vene des Unterarms einer Patientin eingeben kann. Dabei soll eine einfache Steuerung für die automatische zyklische Zugabe flüssiger Arzneimittel verwirklicht werden, die eine einfache Möglichkeit der Einflußnahme, z. B. durch die Patientin, beinhaltet. Darüber hinaus soll die konfektionierte Einheit aus Vorratsbehälter und Schlauch so gestaltet sein, daß sie ggf. von der Patientin einfach und schnell ausgewechselt werden kann, wobei insbesondere der Schlauch einfach in betriebssichere Lage gebracht werden und sich in geschützter Anordnung befinden soll.

Diese Aufgabe wird bei einer Vorrichtung der eingangs genannten Gattung dadurch gelöst, daß der Antriebsmotor mittels der Steuerung im Stundenbereich auf Pausenzeit und im Minutenbereich auf Laufzeit eingestellt ist und daß ein Schaltkontakt zum willkürlichen Einschalten und eine Resettaste zum Einstellen der Steuerung auf Null vorgesehen ist.

Als Zeitgeber für die Impuls- und Pausenzeiten sind vorzugsweise ein Taktgenerator, ein Frequenzteiler und eine Treiberstufe hintereinander geschaltet.

Mit dieser Steuerung wird der Gleichstrom-Antriebsmotor im Stundenbereich auf Pausenzeiten und im Minutenbereich auf Laufzeit eingestellt, so daß beispielsweise alle 90 Minuten eine Einzeldose der Patientin zugegeben wird. Mittels eines speziell vorgesehenen Schaltkontaktes kann der Antriebsmotor willkürlich zu jedem beliebigen Zeitpunkt eingeschaltet werden, während sich die Steuerung durch eine Reset-Taste (Rückstelltaste) auf Null einstellen läßt.

Bei dieser Anordnung läßt sich der Arbeitstakt bzw. die Impulsfolge nicht verändern. Es genügt, daß die Behandlung über verschieden lange Zeiträume erstreckt werden kann.

Gemäß einem weiteren Merkmal der Erfindung weist der über den Kopf der Rollenpumpe geleitete, das flüssige Arzneimittel führende Schlauch zwischen dem Vorratsbehälter und seinem Auslaßende in einem vorgegebenen Abstand voneinander zwei Spannelemente wie Muffen auf, welche hinter Öffnungen einer Wand legbar sind, so daß der Schlauch bzw. das Katheter gespannt um den Kopf der Rollenpumpe gelegt werden kann. Somit erübrigt sich ein mit den einzelnen Rollen des Kopfes der Rollenpumpe zusammenwirkender feststehender Gegenbacken, so daß der Schlauch bzw. das Katheter sehr einfach und schnell eingelegt und auch wieder ausgewechselt werden kann.

Die mit der Erfindung erzielbaren Vorteile bestehen darin, daß der Patientin die vom Arzt verschriebene Menge des flüssigen Arzneimittels, insbesondere des in einem Träger gelösten LH-RH, in dem als natürlich erkannten Takt ambulant eingegeben werden kann. Die Vorrichtung ist am Körper zu tragen und kann immer wieder verwendet werden, weil das Medikament in einem auswechselbaren und gegenüber den anderen Teilen der Vorrichtung gekapselten Behälter-Schlauch-System enthalten ist, so daß keine Bedenken hinsichtlich Sterilität des Medikamentes bestehen.

In der Zeichnung ist ein Ausführungsbeispiel der erfindungsgemäßen Vorrichtung schematisch dargestellt, und zwar zeigt

Fig. 1 eine schaubildliche Ansicht der in einem Gehäuse untergebrachten Vorrichtung,

Fig. 2 eine schaubildliche Ansicht der an einen Gürtel angebrachten tragbaren Vorrichtung in gegenüber Fig. 1 verkleinertem Maßstab und

Fig. 3 ein Schaltbild der Steuerung dieser Vorrichtung.

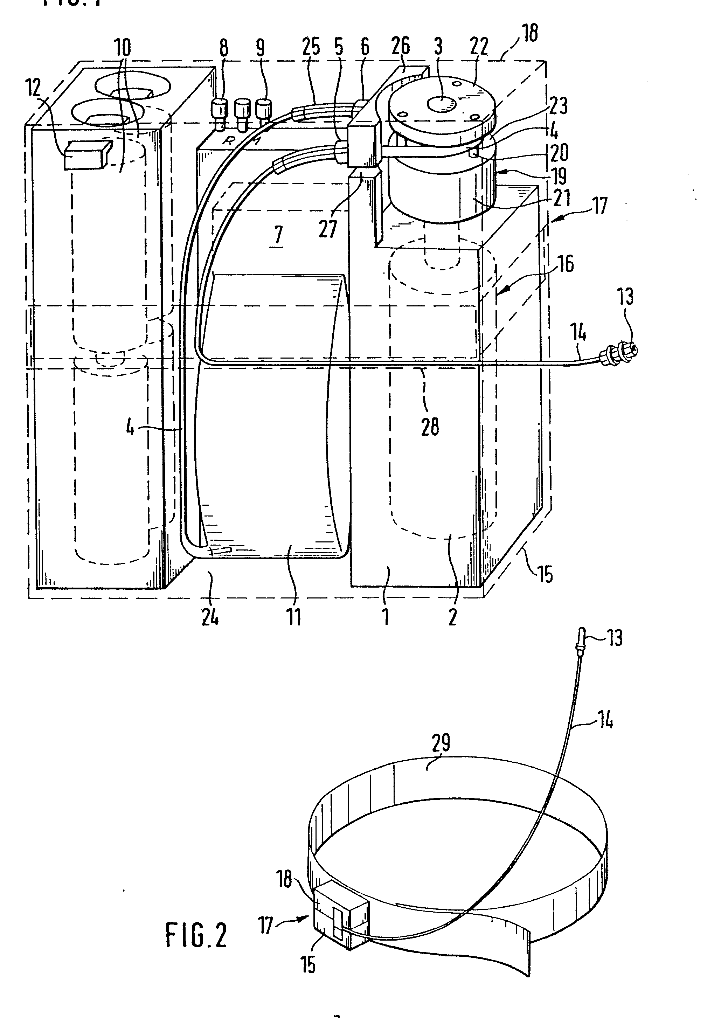

Die Vorrichtung ist in Fig. 1 stark vergrößert dargestellt, um die Einzelteile besser erkennen zu lassen, während die Darstellung in Fig. 2 kleiner als in natürlicher Größe ist.

In einem blockförmigen Rahmen 1 sind eine Rollenpumpe 16, eine elektronische Steuerung 7 für die Rollenpumpe und Gleichstrom liefernde elektrische Batterien 10 als Energiequelle für die Rollenpumpe 16 untergebracht. Der blockförmige Rahmen 1 sitzt im Unterteil 15 eines kastenartigen Gehäuses 17, dessen Deckel 18 abnehmbar ist, so daß der obere Teil des blockförmigen Rahmens 1 frei gelegt werden kann.

Die Rollenpumpe 16 hat einen mit Gleichstrom arbeitenden Antriebsmotor 2, auf dessen Abtriebswelle 3 ein Kopf 19 drehfest angebracht ist, welcher achsparallel zur Abtriebswelle 3 liegende Nadelrollen 20 aufweist, die sich zwischen einer dicken Scheibe 21 und einer dünneren Abdeckscheibe 22 erstrecken, so daß sie in einer Art Führungsschlitz 23 liegen.

In einer Ausnehmung 24 des blockförmigen Rahmens 1 steckt auswechselbar ein in seiner Größe konfektionierter flexibler Vorratsbehälter 11, an den als Auslaß ein Schlauch 4 angeschlossen ist, der durch den Schlitz 23 des Rollenkopfes 19 der Rollenpumpe 16 geführt ist und in einem Katheter 14 mit Anschlußstück 13 endet. Der Schlauch 4 steckt in einer in Fig. 1 nur teilweise dargestellten schlauchförmigen elastischen Hülle 25, auf der in vorgegebenen Abständen als Spannelemente zwei Muffen 5 und 6 befestigt sind. Diese Muffen 5 und 6 legen sich hinter eine am Rahmen 1 vorgesehene hochstehende Wand 26, die mit seitlichen Schlitzen 27 versehen ist, um den elastisch dehnbaren Schlauch 4, 25, einsetzen und dabei gegen die Nadelrollen 20 des Kopfes 19 der Rollenpumpe 16 spannen zu können. Daher wirken im Betrieb die einzelnen Nadelrollen 20 mit dem gespannten Schlauch 4 als Förderelemente zusammen.

Das den Katheter 14 bildende Ende des Schlauches 4 ist in eine Nut 28 auf der Außenseite des Rahmens 1 eingeklemmt, so daß es sich nicht unerwünscht verschieben kann.

Obwohl die schlauchförmige Hülle 25 in Fig. 1 nur in Teilstücken dargestellt ist, sei erwähnt, daß sich diese Hülle nahezu über die gesamte Länge des Schlauches 4 erstrecken kann und somit einen wirksamen Schutz für den sehr feinen Schlauch 4 bildet.

Der Antriebsmotor 2 läuft mit Gleichstrom, welcher von den Batterien 10 über die elektronische Steuerung 7 zugeführt wird. Mit Hilfe eines Druckschalters 8 kann der Antriebsmotor 2 unabhängig von dem vorgegebenen Programm der Steuerung 7 willkürlich jederzeit eingeschaltet werden. Durch Betätigen einer Reset-Taste 9 läßt sich die elektronische Steuerung 7 jederzeit auf Null einstellen.

Ein Kontaktschieber 12 wirkt gegen das obere Ende der vier Batterien 10, wenn die Vorrichtung sich in der Betriebsstellung befindet. Zieht man den vorzugsweise aus Federstahl bestehenden zungenartigen Kontaktschieber 12 heraus, ist der Stromkreis unterbrochen, so daß kein Strom abgegeben wird und die Batterien sich nicht unnötig entleeren können.

Fig. 2 zeigt, daß die Vorrichtung auf der Außenseite eines Gürtels 29 angebracht und dementsprechend problemlos exkorporal von einer Patientin getragen werden kann.

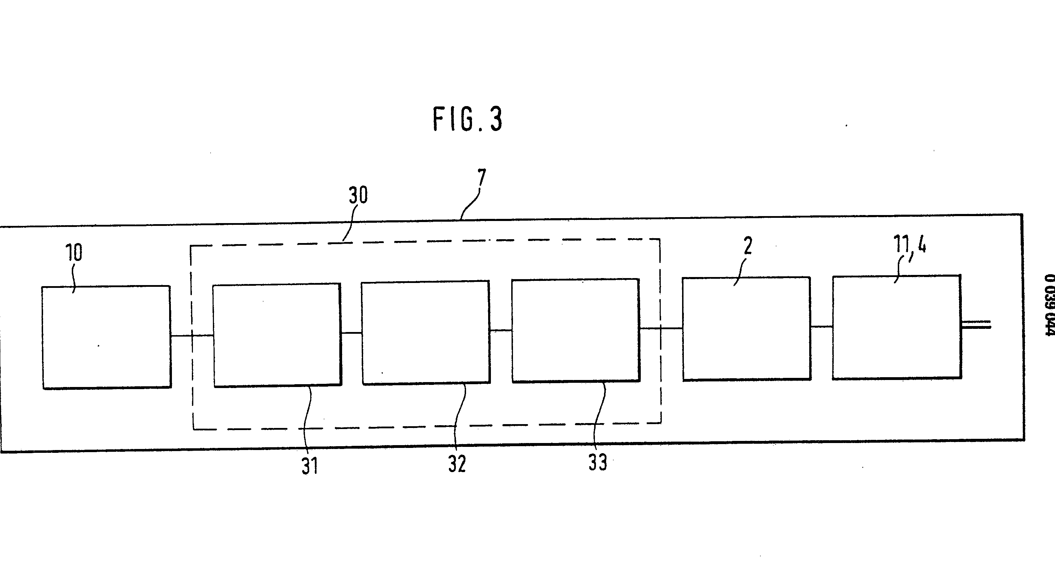

Die elektronische Steuerung 7 enthält, wie Fig. 3 zeigt, einen Zeitgeber 30, welcher Pausenzeiten in Stunden mißt und auf eine bis neunundneunzig Stunden eingestellt werden kann und welcher ferner die Impulszeit mißt, d. h. also die Infusionszeiten, welche auf eine bis neun Minuten eingestellt werden können. Der Zeitgeber enthält in Reihe hintereinander geschaltet einen Taktgenerator 31, einen Frequenzteiler 32 und eine Treiberstufe 33.

Die Vorrichtung arbeitet folgendermaßen:

Zunächst wird ein mit flüssigem Arzneimittel gefüllter Vorratsbehälter 11 in die Ausnehmung 24 eingesteckt und der Schlauch 4 über den Kopf 19 der Rollenpumpe 16 gelegt und festgespannt. Dann drückt man die Reset-Taste 9, um die Elektronik bzw. die Steuerung 7 auf Null einzustellen. Zum Testen der Rollenpumpe 16 wird nun der Druckschalter 8 betätigt. Dadurch wird der Antriebsmotor 2 in Betrieb gesetzt und läuft so lange, wie man auf die Drucktaste 8 drückt. Die Drehung der Abtriebswelle 3 des Antriebsmotors 2 in der Impulszeit entspricht dem Umlauf der Nadelrollen 20, wodurch die im gespannten Teil des Schlauches 4 enthaltene Flüssigkeit in das Katheter 14 und zu dem Anschlußstück 13 gefördert wird, von wo die Flüssigkeit beispielsweise in die Unterarmvene einer Patientin gelangt.

Der Vorratsbehälter 11 bildet mit dem Schlauch 4 und dem Katheter 14 mit Anschlußstück 13 eine konfektionierte sterile Einheit, die nach einmaligem Gebrauch weggeworfen wird. Die mit dem Arzneimittel in Kontakt kommenden Teile bestehen aus Polyäthylen und der Schlauch 4 vorzugsweise aus Silikon. Diese sterile Einheit wird unter Verschluß steril an den Arzt geliefert, der sie in die Vorrichtung einsetzt und die Vorrichtung dann in Betrieb nimmt und der Patientin appliziert.

Das Gehäuse 17 ist beispielsweise rechteckig ausgebildet und hat eine Grundfläche von 100 × 20 mm und eine Höhe von 50 mm. Sie läßt sich deshalb bequem mittels eines Gürtels 29 am Körper tragen.

## Patentansprüche

1. Vorrichtung zur intermittierenden Applikation flüssiger Arzneimittel, mit einem auswechselbaren Vorratsbehälter (11) für das Arzneimittel, einem an den Vorratsbehälter fest angeschlossenen Schlauch (4) zum Abgeben des Arzneimittels, so daß der Vorratsbehälter (11) mit dem Schlauch (4) eine konfektionierte Einheit bildet, einer auf den Schlauch einwirkenden Rollenpumpe (16), einem batteriegetriebenen Antriebsmotor (2) für die Rollenpumpe (16), der mit einer Untersetzung versehen ist, und mit einer Steuerung (7) für den Antriebsmotor (2), die einen Zeitgeber (30) für Impuls- und Pausenzeiten aufweist, dadurch gekennzeichnet, daß der Antriebsmotor (2) mittels der Steuerung (7) im Stundenbereich auf Pausenzeit und im Minutenbereich auf Laufzeit eingestellt ist und daß ein Schaltkontakt (8) zum willkürlichen Einschalten und eine Resettaste (9) zum Einstellen der Steuerung auf Null vorgesehen ist.

2. Vorrichtung nach Anspruch 1, dadurch gekennzeichnet, daß der Zeitgeber (30) der Steuerung (7) einen Taktgenerator (31), einen Frequenzteiler (32) und eine Treiberstufe (33) hintereinandergeschaltet aufweist.

3. Vorrichtung nach Anspruch 1 oder 2, dadurch gekennzeichnet, daß der über die Rollenpumpe (16) gelegte, das flüssige Arzneimittel führende Schlauch (4) zwischen dem Vorratsbehälter (11) und seinem Auslaßende (13) in einem vorgegebenen Abstand voneinander zwei Spannelemente wie Muffen (5, 6) aufweist, welche hinter Öffnungen (27) in einer Wand (26) zum Spannen des Schlauchs auf den Kopf (19) der Rollenpumpe legbar sind, und im Bereich des Kopfes (19) der Rollenpumpe (16) in einer elastischen Schutzhülle (25) untergebracht ist.

## Claims

1. Intermittent delivery system for a medical liquid comprising a removable storage reservoir (11) for the medical liquid, a flexible pipe (4) fastened to said storage reservoir for delivering the medical liquid so that said storage reservoir (11) forms an entitiy with said flexible pipe (4), comprising further a roller pump (16) acting on said flexible pipe, a battery operated drive motor (2) for said roller pump (16) and provided with a reduction gear, and a control mechanism (7) for drive motor (2) comprising a timer (30) for pulse and intermission times, characterized in that drive motor (2) is adjusted by means of control mechanism (7) in the hour range to intermission time and in the minute range to operation time and that there is provided a switching mechanism (8) for optional actuation and a reset button (9) for resetting the control mechanism to zero.

2. System as set forth in claim 1, characterized in that timer (30) of control mechanism (7) comprises connected in series a cycle generator (31), a frequency divider (32) and a driver (33).

3. System as set forth in either of claims 1 or 2, characterized in that the medical liquid conveying flexible pipe (4) provided above roller pump (16) comprises between storage reservoir (11) and its outlet end (13) a pair of clamping elements such as sockets (5, 6) arranged in predetermined distance from one another, which may be disposed behind apertures (27) in a wall (26) for clamping the flexible pipe on top (19) of the roller pump, and that in the area of top (19) of roller pump (16) said flexible pipe is provided with a flexible sleeve (25).

**Revendications**

1. Dispositif de distribution par intermittence d'un liquide médicamenteux comprenant un réservoir détachable (11) pour le liquide médicamenteux, un flexible (4) connecté au réservoir détachable pour distribuer le liquide médicamenteux de sorte que le réservoir détachable (11) forme une entité confectionnée avec le flexible (4), et comprenant une pompe à rouleaux (16) agissant sur le flexible, ainsi qu'un moteur (2) entraîné par batterie pour la pompe à rouleaux (16), ce moteur étant équipé d'une réduction, et comprenant également une commande (7) pour le moteur (2), cette commande comprenant une minuterie (30) pour le réglage des temps d'impulsion et d'arrêt, caractérisé en ce que le moteur (2) est réglé au moyen de la commande (7) dans le domaine horaire au temps d'arrêt et dans le domaine des minutes au temps opératoire et qu'il est prévu un contact de commande (8) pour la mise en service optionnelle et une touche de retour (9) pour la mise à zéro de la commande.

2. Dispositif suivant la revendication 1, caractérisé en ce que la minuterie (30) de la commande (7) comprend en série un générateur de cycles (31), un démultiplicateur de fréquences (32) et un étage excitateur (33).

3. Dispositif suivant l'une des revendications 1 ou 2, caractérisé en ce que le flexible (4) disposé au-dessus de la pompe à rouleaux (16) et transportant de liquide médicamenteux comprend entre le réservoir (11) et sa sortie (13) à une distance prédéterminée l'un de l'autre deux éléments de serrage tels que des manchons (5, 6) pouvant être disposés derrière des ouvertures (27) dans une paroi (26) pour serrer le flexible sur la tête (19) de la pompe à rouleux et étant disposés dans la zone de la tête (19) de la pompe à rouleaux (16) dans une gaine élastique (25).

FIG.1

FIG.2

FIG.3

0 039 044